# EUROPÄISCHE PATENTSCHRIFT

(11) **EP 3 713 549 B1**
(45) Veröffentlichungstag und Bekanntmachung des Hinweises auf die Patenterteilung: **19.06.2024**
(21) Anmeldenummer: 18812087.7
(22) Anmeldetag: 21.11.2018
(51) Int. Cl.: A61K 9/70, A61K 31/506, A61K 31/122, A61K 31/135, A61K 31/381, A61K 31/4468, A61K 31/4545, A61K 31/663

(54) **TRANSDERMALES THERAPEUTISCHES SYSTEM AUF BASIS VON KLEBENDEN WEICHMACHER-POLYMER-MATRICES**
TRANSDERMAL THERPEUTIC SYSTE BASED ON ADHESIVE PLASTICIZER-POLYMER-MATRICES
SYSTEME THERAPEUTIQUE TRANSDERMIQUE A BASE DE PLASTIFIANT-POLYMERE-MATRICES

(30) Priorität: 21.11.2017 DE 102017127433
(43) Veröffentlichungstag der Anmeldung: 30.09.2020
(73) Patentinhaber: LTS Lohmann Therapie-Systeme AG, 56626 Andernach (DE)
(72) Erfinder: LINN, Michael, 55596 Waldböckelheim (DE); MÜLLER, Markus, 53840 Troisdorf (DE); BAUER, Marius, 56626 Andernach (DE)
(74) Vertreter: Meissner Bolte Partnerschaft mbB
(86) Internationale Anmeldenummer: PCT/EP2018/082090
(87) Internationale Veröffentlichungsnummer: WO 2019/110306

(56) Entgegenhaltungen:
- EP-A1- 1 557 164
- EP-A2- 0 195 643
- EP-A2- 0 439 180
- WO-A1-01/47503
- WO-A1-02/08351
- WO-A1-2008/083149
- WO-A1-2011/073998
- WO-A2-03/034900
- WO-A2-2012/103016
- DE-C1- 4 237 453

## Beschreibung

Die vorliegende Anmeldung betrifft ein transdermales therapeutisches System auf Basis von klebenden Weichmacher-Polymer-Matrices, ein Verfahren zu dessen Herstellung und dessen Verwendung als Arzneimittel.

Transdermale therapeutische Systeme (TTS) haben in den letzten Jahren als Darreichungsform zur Behandlung zahlreicher Erkrankungen weite Verbreitung gefunden, da sie gegenüber herkömmlichen Verabreichungsformen mit Vorteilen verbunden sind. Diese bestehen unter anderem in einer präzisen und konstanten Wirkstoffabgabe, die für eine konstante Konzentration des Wirkstoffs im Blutplasma erforderlich ist. Darüber hinaus kann der First-Pass-Effekt umgangen und die Compliance erhöht werden, da der Patient nicht regelmäßig Tabletten einnehmen muss. Ein Vorteil transdermaler therapeutischer Systeme gegenüber anderen topischen Applikationssystemen wie Salben oder Cremes besteht auch darin, dass diese flächengenau und damit dosiergenau appliziert werden können und nicht die Gefahr eines versehentlichen Abwischens der Salbe und der Kontamination anderer Hautstellen besteht.

Transdermale therapeutische Systeme, wie sie aus dem Stand der Technik bekannt sind, umfassen in der Regel eine wirkstoffundurchlässige Rückschicht und eine wirkstoffhaltige, haftklebende Matrixschicht. Die haftklebende Matrixschicht umfasst bei den bekannten transdermalen therapeutischen Systemen mindestens ein haftklebendes Polymer, um das transdermale therapeutische System auf die Haut des Patienten aufzukleben. Diese haftklebenden Polymere, wie sie aus dem Stand der Technik bekannt sind, umfassen in aller Regel haftklebende Polymere auf (Meth)Acrylatbasis und/oder auf Silikonbasis.

Damit weisen die aus dem Stand der Technik bekannten transdermalen therapeutischen Systeme den Nachteil auf, dass der Kompatibilität des pharmazeutisch aktiven Wirkstoffs mit den üblicherweise erhältlichen haftklebenden Polymeren Rechnung getragen werden muss. Dies führt oft zu relativ komplizierten und aufwändigen Formulierungen, da eine mögliche Inkompatibilität zwischen Wirkstoff und den üblichen haftklebenden Polymeren nur durch den Zusatz weiterer Hilfsstoffe, wie beispielsweise weiterer Lösungsmittel und/oder Emulgatoren, überwunden werden kann. Zudem sind Verfahren zur Herstellung von transdermalen therapeutischen Systemen mit solchen komplexen und aufwändigen Formulierungen wirtschaftlich nachteilig. Die WO 02/08351 A1 beschäftigt sich mit medizinischen Haftklebern, deren physikalische und chemische Eigenschaften sowie insbesondere deren Klebrigkeit und plastische Eigenschaften bzw. Kohärenz. Durch Beimischung eines Zusatzstoffes können diese vorteilhaft modifiziert und verbessert werden. Die offenbarten Matrixpolymere sind Cellulosederivate, hauptsächlich Ethylcellulose, Hydroxypropylcellulose und Hydroxypropylmethylcellulose.

Der vorliegenden Erfindung liegt daher die Aufgabe zugrunde, ein transdermales therapeutisches System bereitzustellen, dessen Matrixschicht klebrig genug ist, sodass das System auf die Haut des Patienten aufgeklebt werden kann, aber keine der üblichen haftklebenden Polymere umfasst. Mit dem transdermalen therapeutischen System sollen zudem auch Wirkstoffe verabreichbar sein, die nicht in den üblichen Lösungsmitteln löslich sind, die üblicherweise zusammen mit den üblichen haftklebenden Polymeren verwendet werden. Zudem soll die Matrixschicht des transdermalen therapeutischen Systems vorzugsweise solche Polymere umfassen, die ein besonders breites Kompatibilitätsspektrum mit den verschiedensten pharmazeutisch aktiven Wirkstoffen aufweisen. Die so erhaltenen transdermalen therapeutischen Systeme sollen dabei allerdings ähnlich gute Eigenschaften bezüglich der Wirkstoffpermeation bzw. des Wirkstoffflux wie die herkömmlichen transdermalen therapeutischen Systeme auf Basis von haftklebenden Polymeren aufweisen. Weiterhin soll ein wirtschaftlich akzeptables Herstellungsverfahren für ein derartiges transdermales therapeutisches System bereitgestellt werden.

Die Aufgabe wird erfindungsgemäß durch ein transdermales therapeutisches System gemäß Anspruch 1 gelöst, das eine wirkstoffundurchlässige Rückschicht und eine Polymermatrix auf einer Seite der wirkstoffundurchlässigen Rückschicht umfasst, wobei die Polymermatrix mindestens einen pharmazeutisch aktiven Wirkstoff, mindestens ein an sich nicht haftklebendes Polymer und mindestens einen Weichmacher umfasst, wobei das an sich nicht haftklebende Polymer ein Polyvinylcaprolactam-/Polyvinylacetat-/ Polyethylenglykol-Copolymer, Schellack, ein Vinylpyrrolidon-/Vinylacetat-Copolymer und/oder Polyvinylpyrrolidon umfasst und wobei der mindestens eine Weichmacher Glycerin und/oder Polyethylenglykol umfasst, dadurch gekennzeichnet, dass die Polymermatrix frei von an sich haftklebenden Polymeren ist.

Die Polymermatrix kann auch nur aus den genannten Bestandteilen bestehen.

Insbesondere wurde gefunden, dass durch die Kombination von an sich nicht haftklebenden Polymeren mit Weichmachern eine Polymermatrix erhältlich ist, die haftklebende Eigenschaften aufweist und somit in dem transdermalen therapeutischen System als Haftklebstoff wirken kann. Besonders vorteilhaft ist es, dass Polymere eingesetzt werden können, die mit einer Vielzahl von pharmazeutisch aktiven Wirkstoffen kompatibel sind und dass auf die üblichen Lösungsmittel, die üblicherweise zusammen mit den üblichen haftklebenden Polymeren verwendet werden, verzichtet werden kann. So kann das Kompatibilitätsproblem von pharmazeutisch aktiven Wirkstoffen mit vielen der üblichen an sich haftklebenden Polymeren vermieden werden.

Unter einem an sich haftklebenden Polymer wird ein Polymer verstanden, das an sich als Haftklebstoff, wie in der DIN EN 923:2016-03 definiert, wirken kann. Ein an sich nicht haftklebendes Polymer kann demnach für sich nicht als Haftklebstoff wie vorstehend definiert wirken.

Die Polymermatrix des erfindungsgemäßen transdermalen therapeutischen Systems ist frei von an sich haftklebenden Polymeren.

Insbesondere sind keine haftklebenden Polymere auf (Meth)Acrylat- bzw. Poly(meth)acrylatbasis, Polyisobutylen und/oder haftklebende Polymere auf Silikonbasis und/oder Copolymere davon in dem erfindungsgemäßen transdermalen therapeutischen System enthalten.

Weichmacher sind flüssige oder feste, indifferente organische Substanzen, vorzugsweise mit geringem Dampfdruck, welche ohne chemische Reaktion, vorzugsweise durch ihr Löse- und Quellvermögen, unter Umständen aber auch ohne ein solches, mit hochpolymeren Stoffen in physikalische Wechselwirkung treten und mit diesen ein homogenes System bilden können. Weichmacher verleihen den mit ihnen hergestellten Gebilden bzw. Überzügen bestimmte angestrebte physikalische Eigenschaften, wie z. B. erniedrigte Einfriertemperatur, erhöhtes Formveränderungsvermögen, erhöhte elastische Eigenschaften, verringerte Härte und gegebenenfalls gesteigertes Haftvermögen.

Die wirkstoffundurchlässige Rückschicht ist vorzugsweise inert und möglichst flexibel, so dass das transdermale therapeutische System auch auf ungleichmäßige Hautbereiche aufgetragen werden kann. Für die Rückschicht kann jedes geeignete Material, wie zum Beispiel Polyethylenterephthalat, Polyethylen, Polybutylen, Polyurethan und/oder Polyester, etc., verwendet werden. Vorzugsweise handelt es sich bei der wirkstoffundurchlässigen Rückschicht um eine Polyethylenterephthalatfolie.

Das erfindungsgemäße transdermale therapeutische System umfasst in einer bevorzugten Ausführungsform eine abziehbare Schutzschicht auf der Seite der Matrixschicht auf der sich nicht die wirkstoffundurchlässige Rückschicht befindet. Die abziehbare Schutzschicht kann aus verschiedenen Materialien, wie zum Beispiel Polyethylenterephthalat, Polyethylen und/oder Polypropylen, hergestellt werden und ist auf der mit der wirkstoffhaltigen Polymermatrix in Kontakt stehenden Seite speziell behandelt, um sie möglichst leicht von dieser ablösbar zu machen. Vorteilhafterweise handelt es sich bei der abziehbaren Schutzschicht auf Basis von Polyethylenterephthalat-Schicht.

Das erfindungsgemäße transdermale therapeutische System ist ferner dadurch gekennzeichnet, dass das transdermale therapeutische System keine zusätzliche Klebeschicht, insbesondere auf Basis von haftklebenden Polymeren, auf der Seite der Polymermatrix, auf der sich nicht die wirkstoffundurchlässige Rückschicht befindet, umfasst.

Dies hat den Vorteil, dass auch mögliche Kompatibilitätsprobleme mit dem mindestens einem pharmazeutisch aktiven Wirkstoff und den haftklebenden Polymeren einer zusätzlichen Klebeschicht ausgeschlossen werden können.

Das erfindungsgemäße transdermale therapeutische System ist ferner dadurch gekennzeichnet, dass das an sich nicht haftklebende Polymer ein wasserlösliches Polymer umfasst.

Wasserlösliche Polymere umfassen chemisch sehr unterschiedliche, natürliche oder synthetische Polymere, deren gemeinsames Merkmal ihre Löslichkeit in Wasser oder wässrigen Medien ist. Voraussetzung dafür ist, dass diese Polymere eine für die Wasserlöslichkeit ausreichende Anzahl an hydrophilen Gruppen besitzen und nicht vernetzt sind. Die hydrophilen Gruppen können nicht-ionisch, anionisch, kationisch und/oder zwitterionisch sein.

Das erfindungsgemäße transdermale therapeutische System ist dadurch gekennzeichnet, dass das an sich nicht haftklebende Polymer, ein Polyvinylcaprolactam-/Polyvinylacetat-/ Polyethylenglykol-Copolymer, Schellack, ein Vinylpyrrolidon-/Vinylacetat-Copolymer und/oder Polyvinylpyrrolidon umfasst.

Ein geeignetes Polyvinylcaprolactam-/Polyvinylacetat-/Polyethylenglykol-Copolymer ist beispielsweise unter dem Handelsnamen "Soluplus" von BASF erhältlich. Ein geeignetes Polyvinylpyrrolidon ist beispielsweise unter dem Handelsnamen "Kollidon VA 64" von BASF erhältlich. Diese Polymere haben den Vorteil, dass sie mit einer Vielzahl von pharmazeutisch aktiven Wirkstoffen problemlos kompatibel sind und zudem weitgehend unbedenklich für den Patienten sind.

Das erfindungsgemäße transdermale therapeutische System zeichnet sich dadurch dadurch aus, dass der mindestens eine Weichmacher Glycerin, Polyethylenglykol, insbesondere Polyethylenglykol 200, Sorbitol und/oder Tributylcitrat umfasst.

Besonders bevorzugt umfasst der mindestens eine Weichmacher Glycerin und/oder Polyethylenglykol 200.

Durch den Einsatz einer Polymermatrix, umfassend mindestens ein an sich nicht haftklebendes Polymer und mindestens einen Weichmacher, kann eine haftklebende Polymermatrix bereitgestellt werden, die, bevorzugt nach Trocknen, als Haftklebeschicht in dem erfindungsgemäßen transdermalen therapeutischen System eingesetzt werden kann.

Das erfindungsgemäße transdermale therapeutische System ist bevorzugt dadurch gekennzeichnet, dass die Menge des mindestens einen an sich nicht haftklebenden Polymers in der Matrixschicht etwa 50 bis 90 Gew.-%, bevorzugt etwa 55 bis 85 Gew.-%, besonders bevorzugt etwa 60 bis 80 Gew.-%, bezogen auf das Gesamtgewicht der Matrixschicht, beträgt.

Außerdem ist das erfindungsgemäße transdermale therapeutische System bevorzugt dadurch gekennzeichnet, dass die Menge des mindestens einen Weichmachers in der Matrixschicht etwa 5 bis 50 Gew.-%, bevorzugt etwa 10 bis 30 Gew.-%, bezogen auf das Gesamtgewicht der Matrixschicht, beträgt.

Bevorzugt beträgt das Gewichtsverhältnis in Gewichtsteilen des mindestens einen Polymers zu dem mindestens einen Weichmacher etwa 90 bis 50 zu etwa 10 bis 50 Gewichtsteilen, bevorzugt etwa 85 bis 65 zu etwa 15 zu 35 Gewichtsteilen, besonders bevorzugt etwa 80 bis 60 zu etwa 20 bis 40 Gewichtsteilen.

Wird zu wenig oder zu viel Weichmacher eingesetzt, so ist die Mischung entweder nicht klebrig oder ein verarbeitbarer Masseansatz kann von vorneherein nicht bereitgestellt werden.

Die Auswahl des mindestens einen pharmazeutisch aktiven Wirkstoffs ist grundsätzlich nicht beschränkt, sondern es kann jeder pharmazeutisch aktive Wirkstoff, der zur transdermalen Applikation geeignet ist, eingesetzt werden.

Das erfindungsgemäße transdermale therapeutische System ist bevorzugt dadurch gekennzeichnet, dass der mindestens eine pharmazeutisch aktive Wirkstoff ausgewählt ist aus der Gruppe, bestehend aus Idebenon, Oxybutynin, Riociguat, Rotigotin, Apixaban, Ketamin, Alendronat und/oder Fentanyl.

Bevorzugt beträgt die Menge des mindestens einen pharmazeutisch aktiven Wirkstoffs etwa 1 bis 20 Gew.-%, bevorzugt etwa 5 bis 15 Gew.-%, bezogen auf das Gesamtgewicht der Matrixschicht.

Die Applikationszeit, für die das transdermale therapeutische System vorgesehen ist, beträgt vorzugsweise mindestens etwa 12 Stunden, weiter bevorzugt mindestens etwa 24 Stunden und noch weiter bevorzugt mindestens etwa 48 Stunden. Die Wirkstoffmenge ist entsprechend der gewünschten Applikationszeit abzustimmen.

Das erfindungsgemäße transdermale therapeutische System ist bevorzugt dadurch gekennzeichnet, dass das transdermale therapeutische System mindestens einen Hilfsstoff ausgewählt aus der Gruppe, umfassend Farbstoffe, Emulgatoren, Penetrationsverstärker, pH-Regulatoren, Feuchthaltemittel, Konservierungsmittel und/oder Antioxidationsmittel, vorzugsweise jeweils in einer Menge von 0,01 bis 20 Gew.-%, bezogen auf das Gesamtgewicht der Matrixschicht, umfasst.

Der Penetrationsverstärker ist vorzugsweise ausgewählt aus Fettsäuren und/oder Fettsäureestern, wie Pentansäure, Hexansäure, Octansäure, Nonansäure, Decansäure, Laurinsäure, Myristinsäure, Palmitinsäure, Stearinsäure, Arachidinsäure, Behensäure, Lignocerinsäure, Isovaleriansäure, Neoheptansäure, Neonanonsäure, Isostearinsäure, Ölsäure, Palmitoleinsäure, Linolensäure, Vaccensäure, Petroselinsäure, Elaidinsäure, Oleinsäure, Arachidonsäure, Gadoleinsäure, Erucasäure, Ethylacetat, Methylpropylat, Butylacetat, Methylvalerat, Diethylsebacitat, Methyllaurat, Ethyloleat, Isopropyldecanoat, Isopropylmyristat (Myristinsäureisopropylester), Isopropylpalmitat, Isopropyloleinat (Ölsäure-Isopropyl-Ester), vorzugsweise Ölsäure, Laurinsäure und/oder Myrisitinsäure, besonders bevorzugt Ölsäure, und/oder Fettsäureestern, vorzugsweise Ölsäure-Isopropyl-Ester und/oder Myristinsäureisopropylester.

Das mindestens eine Antioxidans ist vorzugsweise ausgewählt aus alpha-Tocopherol, Ascorbylpalmitat und Butylhydroxytoluol.

Die vorliegende Erfindung betrifft auch ein Verfahren zur Herstellung eines transdermalen therapeutischen Systems, wie vorstehend definiert, umfassend die Schritte
a) Suspendieren des mindestens einen pharmazeutisch aktiven Wirkstoffs in einer Suspension umfassend ein Lösungsmittel auf Basis eines organischen Lösungsmittel und/oder von Wasser, mindestens ein an sich nicht haftklebendes Polymer und mindestens einen Weichmacher,
b) Aufbringen der Suspension erhalten aus a) auf eine wirkstoffundurchlässige Rückschicht und
c) Entfernen des Lösungsmittels.

Das Lösungsmittel, das in Schritt a) eingesetzt wird, ist bevorzugt Wasser.

Die vorliegende Erfindung betrifft ferner ein transdermales therapeutisches System erhältlich durch das vorstehend beschriebene Verfahren.

Die vorliegende Erfindung betrifft auch ein transdermales therapeutisches System wie vorstehend beschrieben oder erhältlich nach dem vorstehend geschilderten Verfahren zur Verwendung als Arzneimittel.

Die im Zusammenhang mit dem erfindungsgemäßen transdermalen therapeutischen System genannten bevorzugten Ausführungsformen gelten auch für das erfindungsgemäße Verfahren und für die erfindungsgemäße Verwendung.

### Beschreibung der Figuren

Figur 1:
   Erfindungsgemäße transdermale therapeutische Systeme zur Verabreichung von Idebenon gemäß den Formulierungen in Tabelle 1. Das linke Diagramm zeigt die kumulative Wirkstoffpermeation, das rechte Diagramm den Wirkstoffflux.
Figur 2:
   Transdermale therapeutische Systeme umfassend einen Haftklebstoff auf Acrylat/Silikon-Basis gemäß dem Stand der Technik. Das linke Diagramm zeigt die kumulative Wirkstoffpermeation, das rechte Diagramm den Wirkstoffflux.
Figur 3:
   Erfindungsgemäße transdermale therapeutische Systeme zur Verabreichung von Oxybutynin gemäß den Formulierungen in Tabelle 1. Das linke Diagramm zeigt die kumulative Wirkstoffpermeation, das rechte Diagramm den Wirkstoffflux.

Die Erfindung wird nachfolgend anhand von nicht beschränkenden Beispielen erläutert.

### Beispiel 1:

Die folgenden Formulierungen wurden erstellt und auf eine Polyethylenterephtalat Folie aufgebracht. Alle Formulierungen weisen haftklebende Eigenschaften auf.

**Tabelle 1: Erfindungsgemäße Formulierungen**

| **Formulierungscode** | **Polymer** | **Weichmacher** | **Wirkstoff** |
|---|---|---|---|
| 124Idb0029 | 80 % Soluplus | 10 % PEG 200 | 10 % Idebenon |
| 13XIdb0011 | 80 % Soluplus | 10 % Glycerin | 10 % Idebenon |
| 13XIdb0012 | 70 % Soluplus | 20 % Glycerin | 10 % Idebenon |
| 13XIdb0009 | 70 % Kollidon VA 64 | 20 % Glycerin | 10 % Idebenon |
| 13XIdb0010 | 60 % Kollidon VA 64 | 30 % Glycerin | 10 % Idebenon |
| 13XObu0001 | 70 % Soluplus | 20 % Glycerin | 10 % Oxybutynin |
| 13XObu0002 | 70 % Soluplus | 20 % PEG 200 | 10 % Oxybutynin |
| 13XObu0003 | 65 % Soluplus | 25 % PEG 200 | 10 % Oxybutynin |
| 13XObu0004 | 60 % Soluplus | 30 % PEG 200 | 10 % Oxybutynin |
| 13XObu0005 | 65 % Kollidon VA64 | 25 % PEG 200 | 10 % Oxybutynin |
| 13XObu0006 | 65 % Kollidon VA 64 | 25 % Glycerin | 10 % Oxybutynin |
| 13XObu0007 | 65 % Kollidon VA 64 | 25 % PEG 200 | 10 % Oxybutynin |
| 13XRio0004 | 60 % Soluplus | 30 % PEG 200 | 10 % Riociguat |
| 13XRio0005 | 60 % Kollidon VA 64 | 30 % Glycerin | 10 % Riociguat |
| 13XRio0006 | 60 % Kollidon VA 64 | 30 % PEG 200 | 10 % Riociguat |
| 13XRio0007 | 60 % Soluplus | 30 % PEG 200 | 10 % Riociguat |
| 13XRio0009 | 60 % Soluplus | 30 % Glycerin | 10 % Riociguat |
| 13XRot0001 | 70 % Soluplus | 20 % Glycerin | 10 % Rotigotin |
| 13XRot0002 | 70 % Kollidon VA 64 | 20 % Glycerin | 10 % Rotigotin |
| 13XRot0003 | 75 % Kolidon VA 64 | 15 % Glycerin | 10 % Rotigotin |

**Tabelle 2: Vergleichsformulierungen in herkömmlichen Haftklebstoffen**

| **Formulierungscode** | **Polymer** | **Wirkstoff** |
|---|---|---|
| 13XIdb0001 | 90 % DuroTak 4098 | 10 % Idebenon |
| 13XIdb0002 | 90 % DuroTak 2516 | 10 % Idebenon |
| 13XIdb0003 | 80 % DuroTak 4098 | 10 % Idebenon |
| 13XIdb0004 | 90 % DuroTak 2353 (80% neutralisiert) | 10 % Idebenon |
| 13XIdb0005 | 90 % DuroTak 2353 | 10 % Idebenon |
| 13XIdb0006 | 90 % Bio-PSA 4207 | 10 % Idebenon |
| 13XIdb0007 | 1. Strich 90 % Bio-PSA 4107 | 10 % Idebenon (im 1.Strich) |
| | 2. Strich 10% Enhancermix (35% Miglyol, 25% Dimethylisosorbid, 25% Eucalyptol 15 % n-Dodecanol) Bio-PSA 4207 | |
| 13XIdb0008 | 92,5% Bio-PSA 4602 | 7.5 % Idebenon |

| | | |
|---|---|---|
| DuroTak: Haftklebstoffe auf Basis von Acrylat-Copolymeren (Fa. Henkel) Bio-PSA: Haftklebstoffe auf Basis von Silikon (Fa. Dow Corning) Miglyol: Mittelkettige Triglyceride | | |

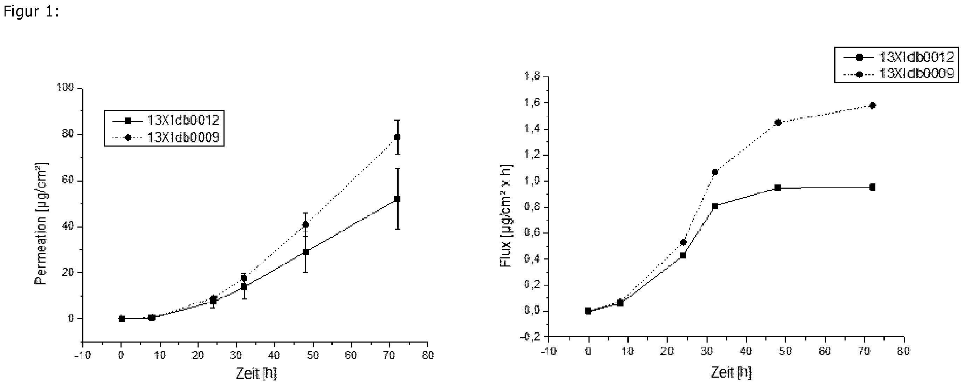

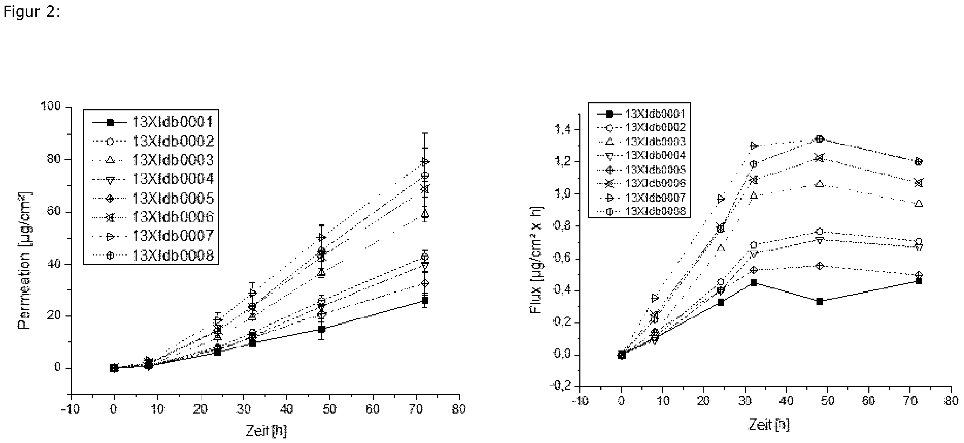

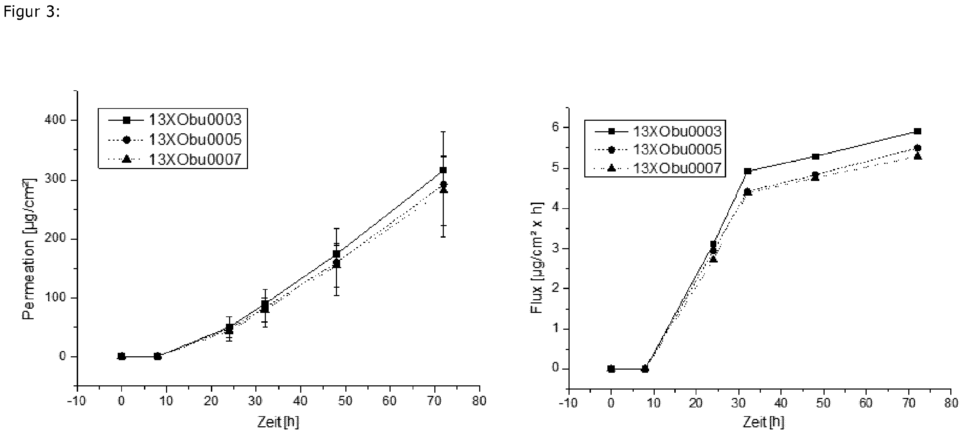

Die *in vitro* Humanhautpermeation einiger in Beispiel 1 aufgeführter Systeme wurde mit Hilfe einer Franz-Zelle gemessen. Im Donor-Kompartiment befindet sich die Substanz bzw. Formulierung (z.B. Gele, Salben, Lösungen, Pflaster). Das Akzeptor-Kompartiment ist mit Puffer oder anderen Lösungen gefüllt. Durch regelmäßige Probennahme aus dem Akzeptor-Kompartiment kann die Permeation einer Substanz über den gewählten Zeitraum durch die Haut hindurch verfolgt werden. Die Verwendung der Franz-Zelle als Diffusionsmodell ist vor allem geeignet den Transport von Arzneistoffen durch humane Haut (= Permeation) vorherzusagen, was der systemischen Verfügbarkeit entspricht. Dabei ist es wichtig zu beachten, dass es keine in-vitro in-vivo Korrelation gibt. Hierbei wurde die Franz-Zelle mit menschlicher Bauchhaut erhalten aus Operationen beschickt. Hierbei wurden 500 µm dermatomisierte Haut mit einer Diffusionsfläche von 1,165 cm² mit dem transdermalen therapeutischen System inkubiert. Als Akzeptormedium diente ein wässriger isotonischer Phosphatpuffer pH = 7,4 plus 0,1% Na-azid mit einem Füllvolumen von 10 mL. Die Messung der Permeation wurde bei einer Temperatur von 32°C durchgeführt und nach 3, 6, 8 und 24 Stunden (n=3) gemessen und ist den Figuren 1 bis 3 zu entnehmen.

Aus den Figuren ist ersichtlich, dass mit den erfindungsgemäßen Formulierungen, die auf klassische Haftkleber verzichten, vergleichbare Ergebnisse gegenüber bekannten Formulierungen hinsichtlich Wirkstoffpermeation und Wirktoffflux erzielt werden können.

## Patentansprüche

1. Transdermales therapeutisches System, umfassend eine wirkstoffundurchlässige Rückschicht und eine Polymermatrix auf einer Seite der wirkstoffundurchlässigen Rückschicht, wobei die Polymermatrix mindestens einen pharmazeutisch aktiven Wirkstoff, mindestens ein an sich nicht haftklebendes Polymer und mindestens einen Weichmacher umfasst, wobei das an sich nicht haftklebende Polymer ein Polyvinylcaprolactam-/Polyvinylacetat-/ Polyethylenglykol-Copolymer, Schellack, ein Vinylpyrrolidon-/Vinylacetat-Copolymer und/oder Polyvinylpyrrolidon umfasst und wobei der mindestens eine Weichmacher Glycerin und/oder Polyethylenglykol umfasst, **dadurch gekennzeichnet, dass** die Polymermatrix frei von an sich haftklebenden Polymeren ist.

2. Transdermales therapeutisches System gemäß Anspruch 1, **dadurch gekennzeichnet, dass** das transdermale therapeutische System keine zusätzliche Klebeschicht auf der Seite der Polymermatrix, auf der sich nicht die wirkstoffundurchlässige Rückschicht befindet, umfasst.

3. Transdermales therapeutisches System gemäß irgendeinem der vorhergehenden Ansprüche, **dadurch gekennzeichnet, dass** die Menge des mindestens einen an sich nicht haftklebenden Polymers in der Matrixschicht etwa 50 bis 90 Gew.-%, bezogen auf das Gesamtgewicht der Matrixschicht, beträgt.

4. Transdermales therapeutisches System gemäß irgendeinem der vorhergehenden Ansprüche, **dadurch gekennzeichnet, dass** das die Menge des mindestens einen Weichmachers in der Matrixschicht etwa 5 bis 50 Gew.-%, bezogen auf das Gesamtgewicht der Matrixschicht, beträgt.

5. Transdermales therapeutisches System gemäß irgendeinem der vorhergehenden Ansprüche, **dadurch gekennzeichnet, dass** der mindestens eine pharmazeutisch aktive Wirkstoff ausgewählt ist aus der Gruppe, bestehend aus Idebenon, Oxybutynin, Riociguat, Rotigotin, Apixaban, Ketamin, Alendronat und/oder Fentanyl.

6. Transdermales therapeutisches System gemäß irgendeinem der vorhergehenden Ansprüche, **dadurch gekennzeichnet, dass** die Menge des mindestens einen pharmazeutisch aktiven Wirkstoffs von etwa 1 bis 20 Gew.-%, bezogen auf das Gesamtgewicht der Matrixschicht, beträgt.

7. Transdermales therapeutisches System gemäß irgendeinem der vorhergehenden Ansprüche, **dadurch gekennzeichnet, dass** das transdermale therapeutische System mindestens einen Hilfsstoff ausgewählt aus der Gruppe, umfassend Farbstoffe, Emulgatoren, Penetrationsverstärker, pH-Regulatoren, Feuchthaltemittel, Konservierungsmittel und/oder Antioxidationsmittel, vorzugsweise jeweils in einer Menge von etwa 0,01 bis 20 Gew.-%, bezogen auf das Gesamtgewicht der Matrixschicht, umfasst.

8. Verfahren zur Herstellung eines transdermalen therapeutischen Systems gemäß irgendeinem der Ansprüche 1 bis 7, umfassend die Schritte:
a) Lösen bzw. Suspendieren des mindestens einen pharmazeutisch aktiven Wirkstoffs in einer Lösung bzw. Suspension umfassend ein Lösungsmittel auf Basis eines organischen Lösungsmittel und/oder von Wasser, mindestens ein an sich nicht haftklebendes Polymer und mindestens einen Weichmacher, wobei das an sich nicht haftklebende Polymer, ein Polyvinylcaprolactam-/Polyvinylacetat-/ Polyethylenglykol-Copolymer, Schellack, ein Vinylpyrrolidon-/Vinylacetat-Copolymer, und/oder Polyvinylpyrrolidon umfasst und wobei der mindestens eine Weichmacher Glycerin und/oder Polyethylenglykol umfasst,
b) Aufbringen der Lösung bzw. Suspension erhalten aus a) auf eine wirkstoffundurchlässige Rückschicht und
c) Entfernen des Lösungsmittels.

9. Transdermales therapeutisches System gemäß irgendeinem der Ansprüche 1 bis 7 zur Verwendung als Arzneimittel.

## Claims

1. A transdermal therapeutic system comprising an active ingredient-impermeable backing layer and a polymer matrix on one side of the active ingredient-impermeable backing layer, wherein the polymer matrix comprises at least one active pharmaceutical ingredient, at least one polymer, which is not pressure-sensitive adhesive itself, and at least one plasticizer, wherein the polymer, which is not pressure-sensitive adhesive itself, comprises a polyvinyl caprolactam-polyvinyl acetate-polyethylene glycol copolymer, shellac, a vinyl pyrrolidone-vinyl acetate copolymer and/or polyvinyl pyrrolidone, and wherein the at least one plasticizer comprises glycerol and/or polyethylene glycol, **characterized in that** the polymer matrix is free of polymers, which are pressure-sensitive adhesive themselves.

2. The transdermal therapeutic system according to claim 1, **characterized in that** the transdermal therapeutic system does not comprise any additional adhesive layer on the side of the polymer matrix on which the active ingredient-impermeable backing layer is not located.

3. The transdermal therapeutic system according to any one of the preceding claims, **characterized in that** the amount of the at least one polymer, which is not pressure-sensitive adhesive itself, in the matrix layer is about 50 to 90% (w/w), based on the total weight of the matrix layer.

4. The transdermal therapeutic system according to any one of the preceding claims, **characterized in that** the amount of the at least one plasticizer in the matrix layer is about 5 to 50% (w/w), based on the total weight of the matrix layer.

5. The transdermal therapeutic system according to any one of the preceding claims, **characterized in that** the at least one active pharmaceutical ingredient is selected from the group consisting of idebenone, oxybutynin, riociguat, rotigotine, apixaban, ketamine, alendronate and/or fentanyl.

6. The transdermal therapeutic system according to any one of the preceding claims, **characterized in that** the amount of the at least one active pharmaceutical ingredient is about 1 to 20% (w/w), based on the total weight of the matrix layer.

7. The transdermal therapeutic system according to any one of the preceding claims, **characterized in that** the transdermal therapeutic system comprises at least one excipient selected from the group comprising colorants, emulsifiers, penetration enhancers, pH regulators, humectants, preservatives and/or antioxidants, preferably each in an amount of about 0.01 to 20% (w/w), based on the total weight of the matrix layer.

8. A method for preparing a transdermal therapeutic system according to any one of the claims 1 to 7, comprising the steps of:
a) dissolving or suspending the at least one active pharmaceutical ingredient in a solution or suspension comprising a solvent based on an organic solvent and/or water, at least one polymer, which is not pressure-sensitive adhesive itself, and at least one plasticizer, wherein the polymer, which is not pressure-sensitive adhesive itself, comprises a polyvinyl caprolactam-polyvinyl acetate-polyethylene glycol copolymer, shellac, a vinyl pyrrolidone-vinyl acetate copolymer and/or polyvinyl pyrrolidone, and wherein the at least one plasticizer comprises glycerol and/or polyethylene glycol,
b) applying the solution or suspension obtained from a) onto an active ingredient-impermeable backing layer, and
c) removing the solvent.

9. The transdermal therapeutic system according to any one of the claims 1 to 7 for use as a medicinal product.

## Revendications

1. Système thérapeutique transdermique, comprenant une couche arrière imperméable au principe actif et une matrice polymère sur un côté de la couche arrière imperméable au principe actif, dans lequel la matrice polymère comprend au moins un principe pharmaceutiquement actif, au moins un polymère non auto-adhésif et au moins un plastifiant, dans lequel le polymère non auto-adhésif comprend un copolymère de polyvinylcaprolactame/polyacétate de vinyle/polyéthylèneglycol, de la gomme-laque, un copolymère de vinylpyrrolidone/d'acétate de vinyle et/ou de la polyvinylpyrrolidone et dans lequel l'au moins un plastifiant comprend de la glycérine et/ou du polyéthylèneglycol, **caractérisé en ce que** la matrice polymère est exempte de polymères auto-adhésifs.

2. Système thérapeutique transdermique selon la revendication 1, **caractérisé en ce que** le système thérapeutique transdermique ne comprend aucune couche adhésive supplémentaire sur le côté de la matrice polymère, sur lequel la couche arrière imperméable au principe actif ne se trouve pas.

3. Système thérapeutique transdermique selon l'une quelconque des revendications précédentes, **caractérisé en ce que** la quantité de l'au moins un polymère non auto-adhésif dans la couche de matrice va d'environ 50 à 90 % en poids par rapport au poids total de la couche de matrice.

4. Système thérapeutique transdermique selon l'une quelconque des revendications précédentes, **caractérisé en ce que** la quantité de l'au moins un plastifiant dans la couche de matrice va d'environ 5 à 50 % en poids par rapport au poids total de la couche de matrice.

5. Système thérapeutique transdermique selon l'une quelconque des revendications précédentes, **caractérisé en ce que** l'au moins un principe pharmaceutiquement actif est choisi parmi le groupe constitué de l'idébénone, de l'oxybutynine, du riociguat, de la rotigontine, de l'apixaban, de la kétamine, de l'alendronate et/ou du fentanyl.

6. Système thérapeutique transdermique selon l'une quelconque des revendications précédentes, **caractérisé en ce que** la quantité de l'au moins un principe pharmaceutiquement actif va d'environ 1 à 20 % en poids par rapport au poids total de la couche de matrice.

7. Système thérapeutique transdermique selon l'une quelconque des revendications précédentes, **caractérisé en ce que** le système thérapeutique transdermique comprend au moins un excipient choisi parmi le groupe comprenant des colorants, des émulsifiants, des renforçateurs de pénétration, des régulateurs de pH, des agents humectants, des agents de conservation et/ou des agents antioxydants, de préférence respectivement en une quantité allant d'environ 0,01 à 20 % en poids par rapport au poids total de la couche de matrice.

8. Procédé de fabrication d'un système thérapeutique transdermique selon l'une quelconque des revendications 1 à 7, comprenant les étapes :
a) de dissolution ou de mise en suspension de l'au moins un principe pharmaceutiquement actif dans une solution ou une suspension comprenant un solvant à base de solvant organique et/ou d'eau, au moins un polymère non autoadhésif et au moins un plastifiant, dans lequel le polymère non autoadhésif comprend un copolymère de polyvinylcaprolactame/polyacétate de vinyle/polyéthylèneglycol, de la gomme-laque, un copolymère de vinylpyrrolidone/d'acétate de vinyle et/ou de la polyvinylpyrrolidone et dans lequel l'au moins un plastifiant comprend de la glycérine et/ou du polyéthylèneglycol,
b) d'application de la solution ou de la suspension obtenue à partir de a) sur une couche arrière imperméable au principe actif, et
c) de retrait du solvant.

9. Système thérapeutique transdermique selon l'une quelconque des revendications 1 à 7 destiné à être utilisé en tant que médicament.
